(19) Europäisches Patentamt
European Patent Office
Office européen des brevets

(11) **EP 2 796 088 A1**

(12) **EUROPEAN PATENT APPLICATION**
published in accordance with Art. 153(4) EPC

(43) Date of publication:
**29.10.2014 Bulletin 2014/44**

(51) Int Cl.:
***A61B 3/024*** (2006.01)

(21) Application number: **12860582.1**

(22) Date of filing: **20.12.2012**

(86) International application number:
**PCT/KR2012/011148**

(87) International publication number:
**WO 2013/094995 (27.06.2013 Gazette 2013/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2011 KR 20110138483**

(71) Applicants:
• **POSTECH ACADEMY-INDUSTRY FOUNDATION
Pohang-si, Gyeongsangbuk-do 790-784 (KR)**
• **Industry Academy Cooperation Foundation of
Kyunghee University
Yongin-si, Gyeonggi-do 446-906 (KR)**

(72) Inventors:
• **YOU, Heecheon
Pohang-si
Gyeongsangbuk-do 790-751 (KR)**

• **LEE, Baekhee
Pohang-si
Gyeongsangbuk-do 790-390 (KR)**
• **LEE, Jihyung
Pohang-si
Gyeongsangbuk-do 790-390 (KR)**
• **KANG, Jaheon
Seoul 135-968 (KR)**

(74) Representative: **Winter, Brandl, Fürniss, Hübner,
Röss, Kaiser, Polte - Partnerschaft mbB
Patent- und Rechtsanwaltskanzlei
Alois-Steinecker-Strasse 22
85354 Freising (DE)**

(54) **PERSONAL-COMPUTER-BASED VISUAL-FIELD SELF-DIAGNOSIS SYSTEM AND VISUAL-FIELD SELF-DIAGNOSIS METHOD**

(57) Provided is a personal computer-based visual field self-testing system of increasing an opportunity of a glaucoma early diagnosis through a self-testing together with improvement of a size reduction, installation, and easiness in movement of a visual field testing system by using a personal computer.

The personal computer-based visual field self-testing system includes a computer monitor presenting a visual field measurement region and a visual field measurement result, an attachable and detachable light blocking unit equipped on a front side of the computer monitor to provide a visual field testing environment, an eye selection unit equipped on a rear side of the attachable and detachable light blocking unit to block external light and select an examination eye, a face support unit including the eye selection unit and supporting a face of an examinee, and a visual stimulus confirmation unit operated by the examinee according to a visual stimulus of the visual field measurement region.

FIG. 1

Description

[Technical Field]

The present disclosure relates to a personal computer-based visual field self-testing system and a visual field self-testing method of measuring a visual field of an examinee by using a personal computer.

[Background Art]

For glaucoma having no current treatment method among various ophthalmological diseases, an early diagnosis is very important. Glaucoma is a disease having an optic nerve atrophy and occurring in an optic nerve including a retinal plexus cell, which is accompanied by a visual field disorder.

A visual field examination provides a clue important to determine a diagnosis and progress of glaucoma by evaluating a function of the optic nerve. A visual field measured through the visual field examination is a viewable range without movement of eyes when the eyes are fixed to one point. It is known that a maximum visual field range of a normal person includes a nasal direction of 60°, a temporal direction of 110°, a superior direction of 60°, and an inferior direction of 75°.

National Health Insurance Service announced that as an analysis result of payment data of health insurance medical expenses, the number of glaucoma disease treatment patients was increased from 207,000 in 2002 to 401,000 in 2009 for 7 years by two times.

In the past, it was not easy to objectively rapidly find damage to the optic nerve. However, recently, in accordance with development of studies of glaucoma diseases and medical apparatus technologies, apparatuses capable of finding damage to the optic nerve in the early stage have been developed and clinically used as compared to the past.

In addition, the number of patients taking a medical examination is increased as an interest in health of ordinary persons is increased. Accordingly, a possibility of finding glaucoma in the early stage by a selective examination is increased.

Accordingly, when an order-made medicine method in which glaucoma is found in the early stage through a regular visual field examination, related knowledge is precisely understood and continuous treatment is performed, and autonomy is provided to diagnosis and treatment methods suitable for individuals is used, a loss of visual field due to glaucoma may be prevented.

Visual field measurement apparatuses are availably used in ophthalmology specialized agencies. However, the visual field measurement apparatuses have a limitation in terms of portability and cost, and excessively satisfy demands of consumers.

First, known visual field measurement apparatuses have a large size and a great weight (e.g.: 600 x 580 x 510 mm, 40 kg; HFA II-i Series). Accordingly, it is known that a predetermined space is required to install the visual field measurement apparatus, and once the visual field measurement apparatus is installed at a position at which light blocking is considered for the purpose of visual field measurement, it is not easy to move the visual field measurement apparatus.

Second, since the known visual field measurement apparatuses are very costly, it is not easy to buy the visual field measurement apparatuses except large-scale ophthalmology specialized agencies (university hospitals and the like). In 2012, it showed that there was no domestic company producing visual field measurement apparatuses. Therefore, it was investigated that domestic ophthalmology disease-relating agencies imported 100% of the visual field measurement apparatus to use even though the visual field measurement apparatus was costly.

Third, all visual field measurement algorithms according to development of a visual field measurement technology are set in the known visual field measurement apparatuses even though a few visual field measurement algorithms are used in practice in the visual field measurement apparatuses. Therefore, the visual field measurement apparatuses need to be constituted to mainly include functions frequently used in an ophthalmology nowadays.

In the visual field examination, a central visual field or a peripheral visual field is examined through visual stimuli arranged in various patterns while a gaze of an examinee is fixed to a central fixation target in a visual field measurement region.

Gaze fixing where the gaze of the examinee is fixed to the central visual target during the visual field examination is an essential process for a precise visual field examination. However, in the known visual field examination apparatuses, whether gaze fixing is performed is confirmed by using an eye camera. Accordingly, a visual field examination method of actively inducing the gaze of the examinee to be fixed needs to be set in the visual field examination apparatuses.

The above information disclosed in this Background section is only for enhancement of understanding of the background of the invention and therefore it may contain information that does not form the prior art that is already known in this country to a person of ordinary skill in the art.

[Disclosure]

[Technical Problem]

The present invention has been made in an effort to provide a personal computer-based visual field self-testing system of increasing an opportunity of a glaucoma early diagnosis through a self-testing together with improvement of a size reduction, installation, and easiness in movement of a visual field testing system by using a personal computer.

Further, the present invention has been made in an effort to provide a gaze fixing method of inducing an examinee to actively concentrate during a visual field examination.

Further, the present invention has been made in an effort to provide a personal computer-based visual field self-testing system connected to a tablet computer to perform a visual field self-testing.

[Technical Solution]

An exemplary embodiment of the present invention provides a personal computer-based visual field self-testing system including a computer monitor connected to a computer main body and presenting a visual field measurement region and a visual field measurement result. An attachable and detachable light blocking unit is equipped on a front side of the computer monitor to provide a visual field testing environment. An eye selection unit is equipped on a rear side of the attachable and detachable light blocking unit to block external light and select an examination eye. A face support unit includes the eye selection unit and supports a face of an examinee. A visual stimulus confirmation unit is connected to the computer main body and operated by the examinee according to a visual stimulus of the visual field measurement region.

The computer monitor may include a first monitor providing the visual field measurement region testing a visual field and a user interface testing the visual field, and a second monitor providing a performed visual field testing result.

The attachable and detachable light blocking unit may come into close contact with the computer monitor at a position completely including the visual field measurement region displayed in the computer monitor, and may block the external light to the computer monitor.

The eye selection unit may include a housing surrounding eyes of the examinee to block the external light, an examination eye body tube fastened to a side of the housing to come into close contact with an eye of the examinee, which is to be examined, a connection unit fastened so that the body tube coincides with a center of the visual field measurement region and connected to a narrow end of the attachable and detachable light blocking unit, and a fence provided at an outskirt of the examination eye body tube in the housing.

The eye selection unit may be supported by a horizontal setting unit provided on a side of the connection unit.

The face support unit may include a forehead support member on which a forehead of the examinee is leaned, a height adjustment member adjusting a height of the forehead support member, a chin support member supporting a chin of the examinee, a left and right adjustment member adjusting left and right movement of the chin support member, a height adjustment screw adjusting a height of the chin support member, and a prop fastened by the height adjustment screw to support a face weight of the examinee.

The visual stimulus confirmation unit may include a left button and a right button pushed by the examinee according to a shape of a central fixation target presented in the visual field measurement region, a visual stimulus confirmation button pushed when the presented visual stimulus is displayed, and a connection port connected to the computer main body.

The visual field measurement region may include a central fixation target, the visual stimuli, and a blind spot visual stimulus constituted to measure a visual field of an eye to be examined.

The central fixation target may be formed to have at least one selected from a numeral, a sign, a letter, a figure, and animal and matter shapes.

The system may further include an examinee information input unit to recognize examinee information constituted by a bar code or a RFID.

The computer monitor may be a tablet computer integrated with the computer main body. In this case, the system may further include a device insertion unit into which the tablet computer is inserted, and a device angle adjustment unit connected to a lower end of the device insertion unit to lean the tablet computer. The visual stimulus confirmation unit may be connected to the tablet computer.

The visual field measurement region may be constituted to selectively perform an ophthalmology examination including visual acuity, macular degeneration, and color blindness.

Another exemplary embodiment of the present invention provides a personal computer-based visual field self-testing method including an input step of inputting a visual field measurement region, a visual field measurement method, and a central fixation target shape in a computer main body, a visual stimulus presentation step of simultaneously performing presentation of a central fixation target and presentation of a visual stimulus or a blind spot visual stimulus, a first judging step of judging whether an examination of the visual stimulus is finished and performing the visual stimulus presentation step when the examination of the visual stimulus is not finished, a gaze fixing error rate calculation step of calculating a gaze fixing error rate when the examination of the visual stimulus is finished, a second judging step of judging whether the gaze fixing error rate is a set value or less, and performing the input step when the gaze fixing error rate is more than the set value, and a result provision step of presenting a visual field measurement result to a computer monitor and finishing a visual field testing program when the gaze fixing error rate is the set value or less.

The input step may further include a conversion step of automatically grasping a size and a resolution of the computer monitor and converting the visual field measurement region to be the same as a screen of the computer monitor and thus display the converted visual field measurement region.

The visual stimulus presentation step may include presenting a plurality of central fixation targets having different shapes by flickering at the same or different speeds.

The result provision step may further include presenting a degree of visual field damage progress based on the individually accumulated visual field measurement result.

According to the exemplary embodiment of the present invention, there is an effect of an increase in opportunity of a glaucoma early diagnosis through a self-testing together with improvement of a size reduction, installation, and easiness in movement of a visual field testing system by using a personal computer. According to the exemplary embodiment of the present invention, there is an effect of improvement of examination concentration and a reduction in examination time by allowing an examinee to actively fix a gaze during a visual field examination.

[Description of Drawings]

FIG. 1 is an entire schematic diagram of a personal computer-based visual field self-testing system according to an exemplary embodiment of the present invention.

FIG. 2 is a perspective view of an attachable and detachable light blocking unit applied to the system of FIG. 1.

FIG. 3 is a perspective view of an eye selection unit applied to the system of FIG. 1.

FIG. 4 is a perspective view of a face support unit applied to the system of FIG. 1.

FIG. 5 is a perspective view of a visual stimulus confirmation unit applied to the system of FIG. 1.

FIG. 6 is a constitutional diagram of a visual field measurement region of a visual field testing program applied to the system of FIG. 1.

FIG. 7 is a flowchart of a visual field self-testing method according to the exemplary embodiment of the present invention.

FIGS. 8A to 8C are constitutional diagrams of a central visual stimulus applied to the visual field self-testing method of FIG. 7.

FIG. 9 is a constitutional diagram in which the same visual field measurement region is provided in the computer monitor of FIG. 1.

FIG. 10 is an entire schematic diagram of a visual field self-testing system with which a tablet computer is connected according to another exemplary embodiment of the present invention.

[Mode for Invention]

The present invention will be described more fully hereinafter with reference to the accompanying drawings, in which exemplary embodiments of the invention are shown. As those skilled in the art would realize, the described embodiments may be modified in various different ways, all without departing from the spirit or scope of the present invention. The drawings and description are to be regarded as illustrative in nature and not restrictive. Like reference numerals designate like elements throughout the specification.

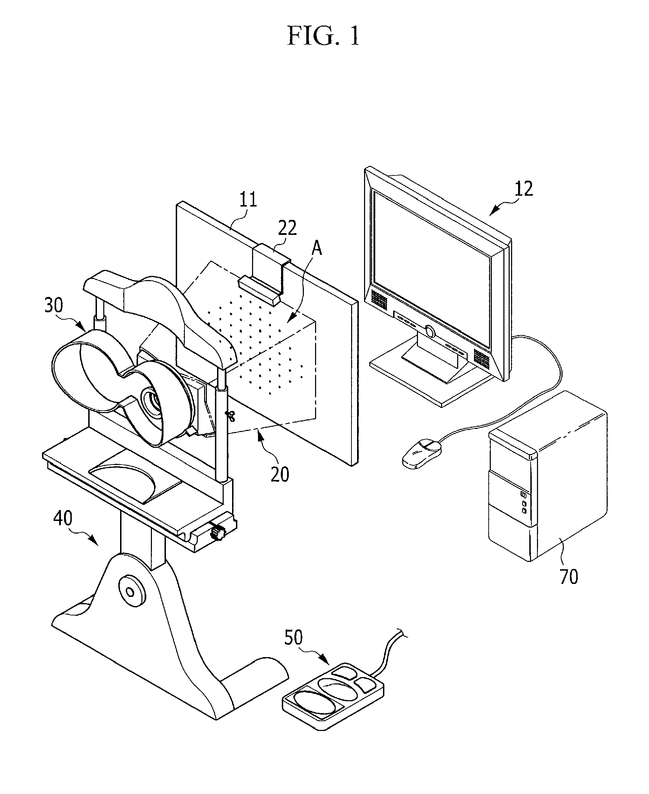

FIG. 1 is an entire schematic diagram of a personal computer-based visual field self-testing system according to an exemplary embodiment of the present invention. Referring to FIG. 1, the personal computer-based visual field self-testing system (hereinafter, referred to as "system") of the exemplary embodiment includes a first monitor 11 presenting a visual field measurement region A testing a visual field, a second monitor 12 presenting a visual field measurement result, an attachable and detachable light blocking unit 20 providing a visual field testing environment, an eye selection unit 30, a face support unit 40, and a visual stimulus confirmation unit 50. The first monitor 11, the second monitor 12, and the visual stimulus confirmation unit 50 are connected to a computer main body 70.

The first monitor 11 shows the visual field measurement region A testing the visual field and a user interface testing the visual field. The second monitor 12 shows a visual field testing result performed through the first monitor 11 in real time. The first and second monitors 11 and 12 are connected to the computer main body 70. When only one computer monitor is used, the visual field measurement region and the visual field measurement result may be sequentially presented to one first monitor 11 (not shown).

The attachable and detachable light blocking unit 20 comes into close contact with the first monitor 11 by using a latch 22 or tongs (not shown) at a position completely including the visual field measurement region A displayed in the first monitor 11. For example, the latch 22 may be formed to be bent in a "Π" form. The attachable and detachable light blocking unit 20 is formed to come into close contact with the first monitor 11 to block external light when an examinee sees the visual field measurement region A.

The eye selection unit 30 is formed to cover a gaze of an opposite eye of an eye to be examined and thus examine a single eye of the examinee. The eye selection unit 30 is connected to block external light at an end of the attachable and detachable light blocking unit 20.

The face support unit 40 is connected to the eye selection unit 30 to fix a face of the examinee. The face support unit 40 comes into close contact with a forehead and a chin of the examinee to fix a face portion, and may be adjusted in upward, downward, left, and right directions to correspond to a shape of the face portion of the examinee. Further, a height of the face support unit 40 may be adjusted in upward and downward directions to fix the face while the examinee feels comfortable.

The visual stimulus confirmation unit 50 includes a left button 52 and a right button 53 selectively pushed by the examinee according to a shape of a central fixation target presented in the visual field measurement region A, and a visual stimulus confirmation button 51 sequentially pushed when a visual stimuli 62 presented at the same time while the left and right buttons 52 and 53 are pushed is displayed. For example, the visual stimulus confirmation unit 50 may be connected to the computer main body 70 through a connection port 55 (for example, a USB port). The central fixation target may be formed to have at least one selected from a numeral, a sign, a letter, a figure, and animal and matter shapes.

The visual field measurement region A is presented in the first monitor 11. The examination visual stimuli 62 are presented by a visual field testing program algorithm of embodying a gaze fixing method by which the visual field of the examinee is measured. The visual field testing program algorithm is set in the computer main body 70. The same visual field measurement region application method is applied to the visual field measurement region A to present the same visual field measurement region regardless of a size and a resolution of the first monitor 11. A central fixation target 61 at the center of the visual field measurement region A is presented to have a shape to which the gaze of the examinee is actively fixed.

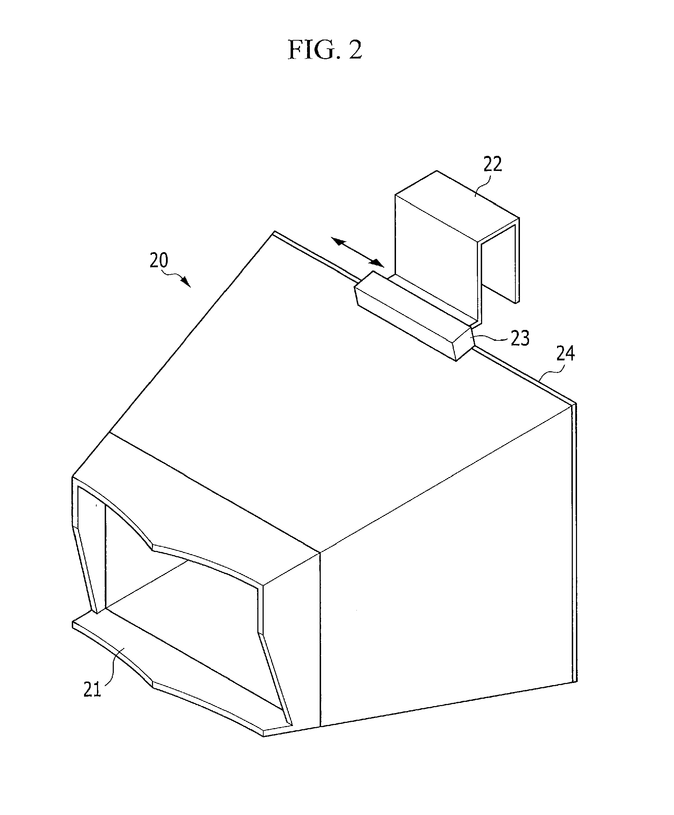

FIG. 2 is a perspective view of the attachable and detachable light blocking unit 20 applied to the system of FIG. 1. Referring to FIGS. 1 and 2, the attachable and detachable light blocking unit 20 is constituted to form a quadrangular pyramid having a cut upper portion and opened both ends. The attachable and detachable light blocking unit 20 has a folding structure that can be folded or unfolded according to an edge.

A wide end 24 of the attachable and detachable light blocking unit 20 is opened to have a size completely including the visual field measurement region A presented in the first monitor 11, and attached to the first monitor 11.

Further, the wide end 24 of the attachable and detachable light blocking unit 20 is constituted by a material such as a rubber or a gel to come into close contact with the first monitor 11 and thus completely block external light. A narrow end 21 of the attachable and detachable light blocking unit 20 is a portion firmly connected to the eye selection unit 30.

The wide end 24 of the attachable and detachable light blocking unit 20 includes a latch holder 23. The latch 22 is fixed to the latch holder 23 forming a groove. The latch 22 may be inserted into the groove of the latch holder 23 in a side direction and move along a width of the first monitor 11.

A length of the latch 22 may be vertically adjusted in consideration of a height of the attachable and detachable light blocking unit 20 completely including the visual field measurement region A (not shown). The latch 22 is flexible but constituted by a material such as iron and reinforced plastics having strength. Accordingly, the first monitor 11 may be fixed according to a shape of an upper end of the first monitor 11.

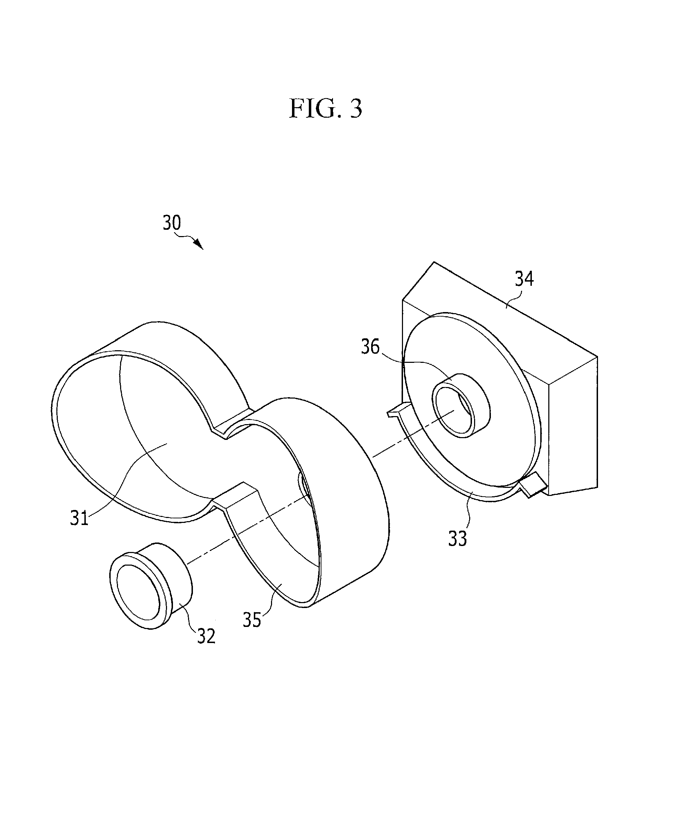

FIG. 3 is a perspective view of the eye selection unit 30 applied to the system of FIG. 1. Referring to FIGS. 1 and 3, the eye selection unit 30 is constituted to examine the single eye of the examinee. The eye selection unit 30 is constituted to have a shape that is similar to that of binoculars or goggles.

The eye selection unit 30 includes a housing 35 surrounding eyes of the examinee to block external light, an examination eye body tube 32 provided in the housing 35 to come into close contact with the eye of the examinee, which is to be examined, and a connection unit 34 fastened to the examination eye body tube 32.

The examination eye body tube 32 is fastened through a through hole of the housing 35 of the eye selection unit 30 to a shaft unit 36 provided at the center of the connection unit 34 so that the examination eye body tube 32 coincides with the center of the visual field measurement region A. Accordingly, the housing 35 may rotate based on the examination eye body tube 32 and the shaft unit 36 fastened to each other.

A horizontal setting unit 33 is provided at a lower side of the shaft unit 36 in the connection unit 34. The horizontal setting unit 33 has a curved surface corresponding to an outskirt of the housing 35 to support the outskirt of the housing 35. The connection unit 34 is connected to the narrow end 21 of the attachable and detachable light blocking unit 20.

Further, subsequently, when the eyes of the examinee come into close contact with the examination eye body tube 32, the examination eye body tube 32 and the first monitor 11 are positioned at a position at which blind spot visual stimuli 63 and 64 of the visual field measurement region A are not confirmed.

In the eye selection unit 30, the housing 35 is covered by a fence 31 with the exception of the examination eye body tube 32. That is, when the examinee sees the visual field measurement region A while the eye to be examined comes into contact with the examination eye body tube 32, a gaze of an opposite eye is covered by the fence 31.

FIG. 3 shows an example of the case where a right eye is examined. When a left eye is examined, the housing 35 of the eye selection unit 30 rotates toward an opposite side along a curved surface of the horizontal setting unit 33. When a central portion of the housing 35 of the eye selection unit 30 is put on the end of the horizontal setting unit 33, the eye selection unit 30 is maintained in a horizontal state where the left eye can be examined.

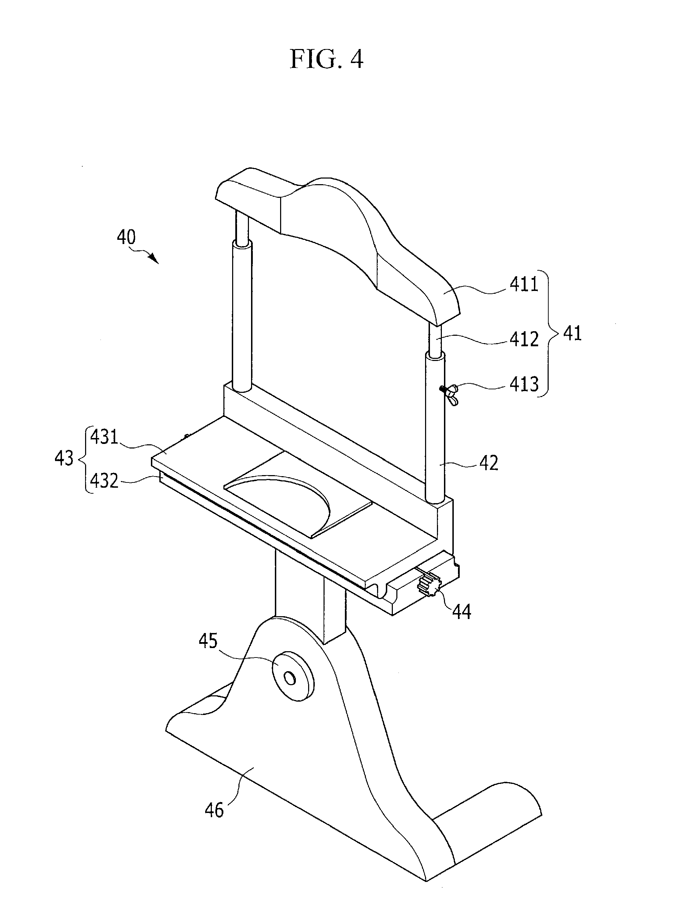

FIG. 4 is a perspective view of the face support unit 40 applied to the system of FIG. 1. Referring to FIGS. 1 and 4, the face support unit 40 includes a forehead support member 41 on which a forehead of the examinee is leaned, a height adjustment member 42 adjusting a height of the forehead support member 41 according to a face length of the examinee, a chin support member 43 supporting a chin of the examinee, a left and right adjustment member 44 designed to move the chin support member 43 in left and right directions according to the examined eye, a height adjustment screw 45 designed to adjust a height of the chin support member 43 according to a face height of the examinee, and a prop 46 supporting a face weight of the examinee.

For example, the forehead support member 41 includes a horizontal member 411 supporting the forehead and a vertical member 412 connected to the horizontal member 411. The height adjustment member 42 is formed of a tube body fastened to the vertical member 412. An adjusted height of the vertical member 412 may be maintained by a stop screw 413 screwed to the height adjustment member 42.

The chin support member 43 includes an upper member 431 having the height adjustment member 42 and formed to support the chin, and a lower member 432 combined so that the left and right adjustment member 44 is interposed between the upper member and the lower member. Accordingly, the upper member 431 may move in left and right directions according to a guide of the left and right adjustment member 44 on the lower member 432.

The height adjustment screw 45 connects the chin support member 43 and the prop 46 to adjust the height of the chin support member 43. The lower member 432 is fastened to the prop 46 so that the lower member 432 ascends and descends. The lower member 432 is screwed by the height adjustment screw 45 (not shown). The lower member 432 ascends and descends according to an operation of the height adjustment screw 45.

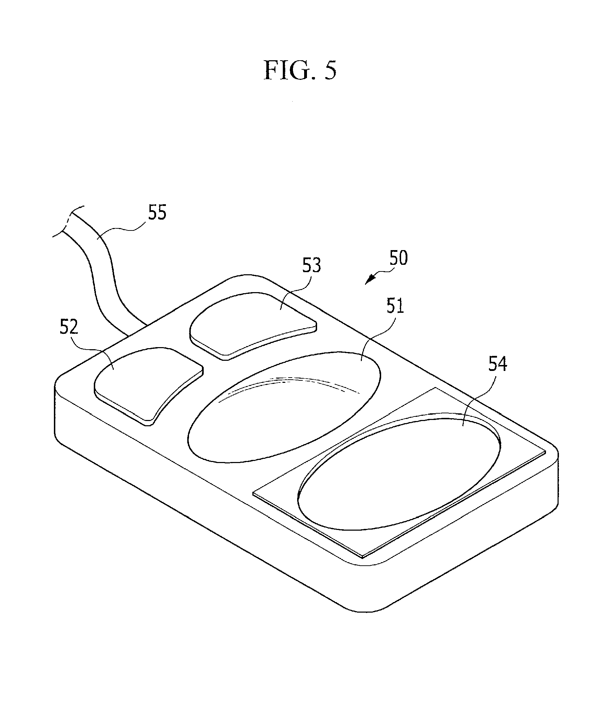

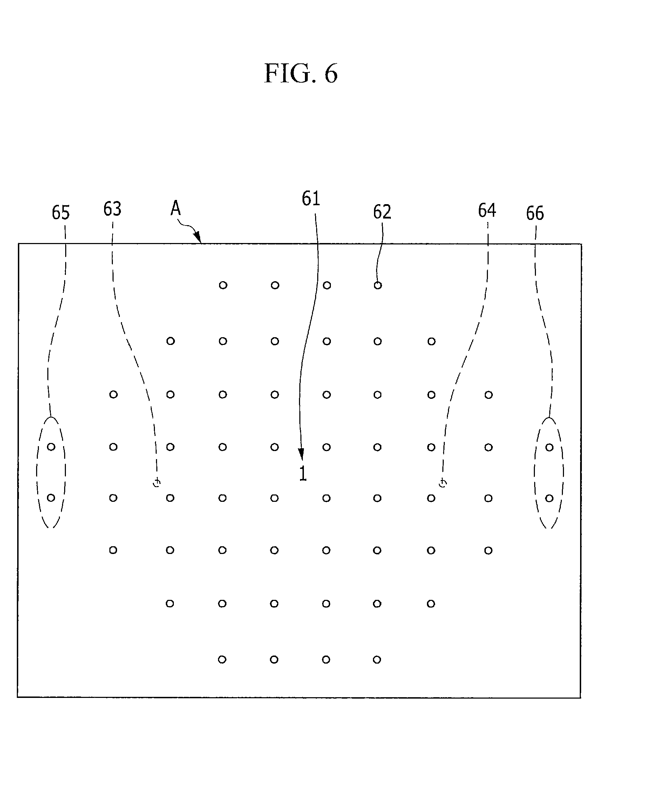

FIG. 5 is a perspective view of the visual stimulus confirmation unit 50 applied to the system of FIG. 1. FIG. 6 is a constitutional diagram of the visual field measurement region A of a visual field testing program applied to the system of FIG. 1.

Referring to FIGS. 1, 5, and 6, when the central visual stimulus 61 is presented as numerals of '1' and '2' during the examination, the examinee confirms whether the central visual stimulus 61 in the visual field measurement region A is '1' or '2'. When the central visual stimulus 61 is '1', the examinee pushes the left button 52, and when the central visual stimulus 61 is '2', the examinee pushes the right button 53. When the examinee sees the visual stimulus 62 presented at the same time as the central visual stimulus 61, the examinee sequentially pushes the visual stimulus confirmation button 51.

The visual stimulus confirmation unit 50 further includes a prop 54 on which a palm is put for the purpose of comfortability of a wrist of the examinee. A USB port 55 is connected to the computer main body 70 to use the visual stimulus confirmation unit 50.

The visual stimulus confirmation unit 50 may be replaced by a voice recognition unit. In the voice recognition unit, confirmation of the central fixation target, the visual stimulus, and the blind spot may be inputted through a voice.

The system may further include an examinee information input unit to recognize examinee information constituted by a bar code or a RFID.

Referring to FIG. 6, the visual field measurement region A includes the central visual stimulus 61, the visual stimulus 62, and the blind spot visual stimuli 63 and 64. The visual stimulus 62 are positioned at points at which predetermined viewing angles from the examination eye body tube 32 of the eye selection unit 30 are formed in the visual field measurement region A.

For a visual field testing, the visual stimulus 62 or the blind spot visual stimuli 63 and 64 are presented at the same time as the central visual stimulus 61, the examinee pushes the left button 52 or the right button 53 of the visual stimulus confirmation unit 50, which is designed to correspond to the confirmed shape of the central visual stimulus 61. When the examinee sees the presented visual stimulus 62, the examinee sequentially pushes the visual stimulus confirmation button 51 of the visual stimulus confirmation unit 50.

For the blind spot visual stimuli 63 and 64, when the left eye of the examinee is examined, only the left blind spot visual stimulus 63 is presented. When the right eye is examined, only the right blind spot visual stimulus 64 is presented. When the visual stimulus 62 is presented during the examination, in consideration of the visual field measurement region A of each eye, in the case where the left eye is examined, two visual stimuli 65 at an end of a left side are not presented. In the case where the right eye is examined, two visual stimuli 66 at an end of a right side are not presented. In the visual field measurement region A, when the blind spot visual stimuli 63 and 64 are presented at an invisible position when the eye of the examinee comes into close contact with the examination eye body tube 32.

An interval between the visual stimulus 62 shown in FIG. 6 is set so that the same viewing angle is formed in left, right, upward, and downward directions when the eye of the examines comes into close contact with the examination eye body tube 32.

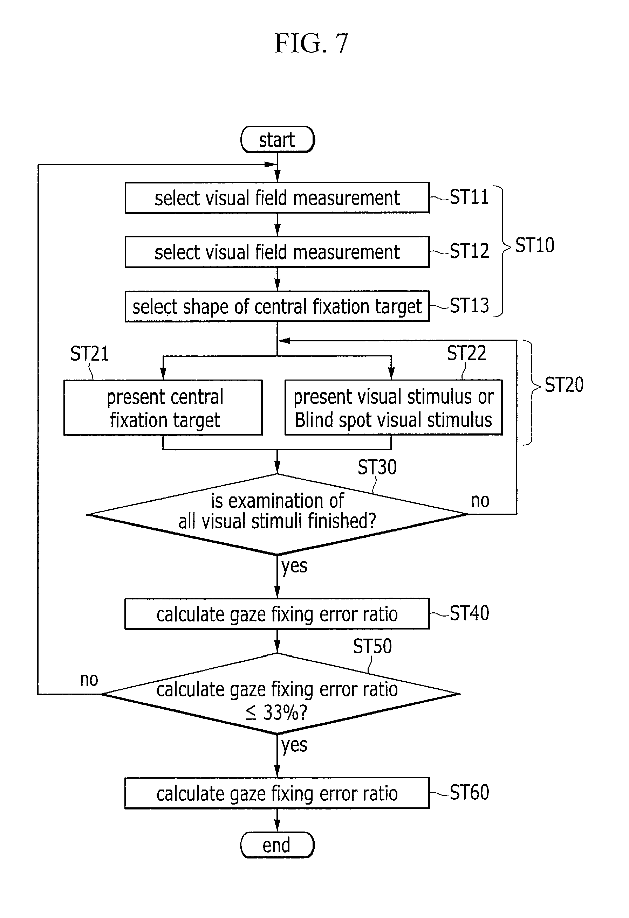

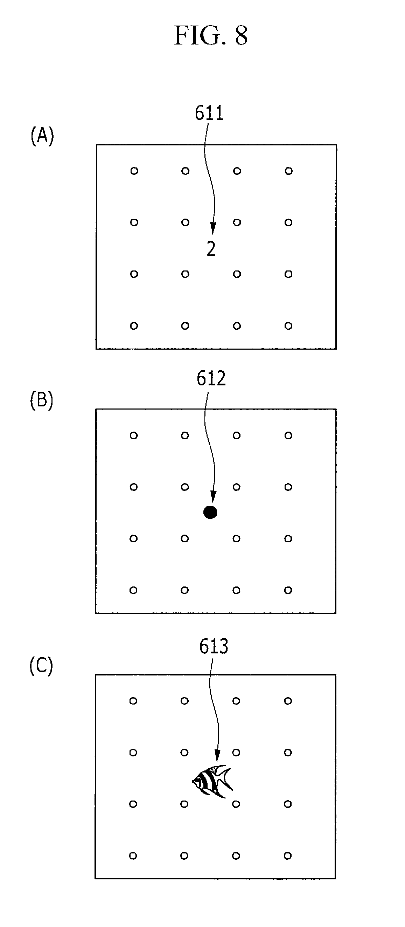

FIG. 7 is a flowchart of a visual field self-testing method according to the exemplary embodiment of the present invention. FIGS. 8A to 8C are constitutional diagrams of a central visual stimulus applied to the visual field self-testing method of FIG. 7.

Referring to FIG. 8, precise examination is performed only when a gaze of the examinee is fixed to central fixation targets 611, 612, and 613 during the visual field examination. That is, the examinee may actively see the central fixation targets 611, 612, and 613. For example, in the exemplary embodiment of the present invention, numeral "2" 611, a colored figure 612, an animal or matter shape 613, and the like are exemplified.

When the gaze of the examinee is not fixed to the central fixation target 61, the examinee cannot see the numeral "2" 611, the colored figure 612, the animal or matter shape 613, and the like presented during a very short period. Accordingly, the examinee cannot precisely push the left button 52 or the right button 53 of the visual stimulus confirmation unit 50.

Accordingly, the gaze of the examination eye of the examinee is actively fixed to the central fixation targets 611, 612, and 613.

The system of the present exemplary embodiment may include a voice information function to perform a self-testing.

Referring to FIGS. 7 and 8, the visual field self-testing method according to the exemplary embodiment includes an input step ST10, a visual stimulus presentation step ST20, a first judging step ST30, a gaze fixing error rate calculation step ST40, a second judging step ST50, and a result provision step ST60.

In the input step ST10, the visual field measurement region A, a visual field measurement method, a central fixation target shape, and examinee information are inputted through a user interface to the computer main body 70. Examinee information may be inputted through an examinee information input unit by using a bar code or a RFID. A name, a sex, and an age may be inputted as essential examination information. Information such as visual acuity, a cornea thickness, intraocular pressure, a cup-disc ratio, and blood pressure may be inputted as additional information. Additional information may be used to more precisely diagnose glaucoma.

In a visual field measurement region A input ST11, before the visual stimuli are presented to the visual field measurement region A, the visual field measurement region required by the examinee is inputted. For example, there is a central visual field or a peripheral visual field in the visual field measurement region.

In a visual field measurement method input ST12, a set visual stimulus presentation interval is inputted. The visual stimulus presentation interval may be determined as a constant or arbitrary interval. In general, when the visual stimulus presentation interval is constant (for example, the interval of 1 sec), the examinee has a temporary habit of pushing the confirmation button at a constant speed. Accordingly, even though the visual stimulus is not visible, the examinee may wrongly think that he/she saw the visual stimulus due to the temporary habit to push the confirmation button by mistake. Accordingly, when the visual stimulus presentation interval is arbitrarily set, the habit of pushing the confirmation button at the constant speed may be minimized to obtain a more precise examination result. In a central fixation target shape input ST13, the numeral or the colored shape is inputted.

In the visual stimulus presentation step ST20, a step SR 21 of presenting the central visual stimulus 61 and a step ST22 of presenting the visual stimulus 62 or the blind spot visual stimuli 63 and 64 are simultaneously performed.

In the first judging step ST30, whether an examination of all visual stimuli is finished is judged. When the examination of the visual stimuli are not finished, the first judging step proceeds to the fixation target and blind spot visual stimulus presentation step ST20. When the examination is finished, the first judging step proceeds to the gaze fixing error rate calculation step ST40.

A gaze fixing error rate calculated in the gaze fixing error rate calculation step ST40 may be represented by a ratio of the number of blind spot visual stimuli 63 and 64 reacted to be seen by the examinee to the given number of blind spot visual stimuli 63 and 64.

In the second judging step ST50, whether the gaze fixing error rate is a set value or less is judged. When the gaze fixing error rate is more than the set value (for example, 20%), reliability of the visual field measurement examination is low. Accordingly, a reexamination from the input step ST10 is performed.

In the result provision step ST60, when the gaze fixing error rate is the set value (for example, 20%) or less, the visual field measurement result is provided to the second monitor 12, and the visual field testing program is finished.

A user interface of the visual field measurement result provides the examination result represented by the numeral and the color according to the degree of visual field damage to the visual field measurement region. Accordingly, an ordinary person can easily understand. The visual field measurement result may be remotely transferred to a medical specialist. The visual field measurement result may be individually stored in an internet-based server to measure the degree of visual field damage progress.

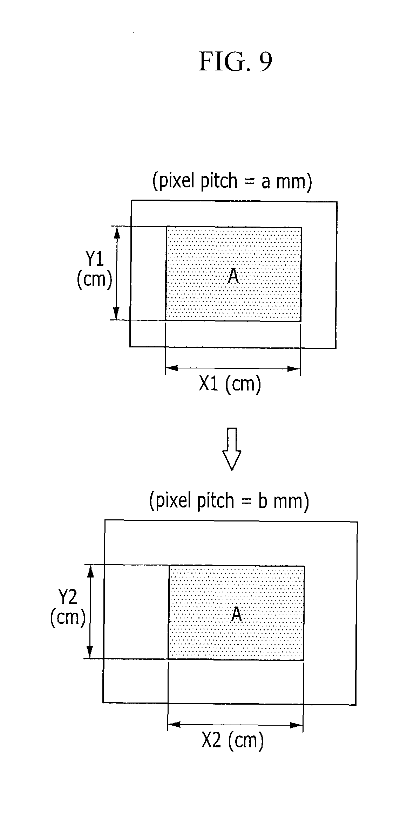

FIG. 9 is a constitutional diagram in which the same visual field measurement region is provided in the computer monitor of FIG. 1.

In the system of the exemplary embodiment, the attachable and detachable light blocking unit 20 is constituted to completely include the visual field measurement region A and come into close contact with a screen of the first monitor 11 regardless of a size and a resolution of the first monitor 11.

The visual field testing program automatically grasps information of the size and the resolution of the first monitor 11 to display the visual field measurement region A having the same size as the screen of the first monitor 11.

The visual field measurement region A input ST11 further includes a conversion step of converting the visual field measurement region to have the same size as the first monitor.

For example, a pixel pitch of the first monitor 11 is a (mm), and a length and a width are X1 and Y1 (cm). Like Equations 1 and 2, in the conversion step, when the visual field measurement region of the first monitor (left) having the pixel pitch of a (mm) is converted into that of the first monitor (right) having a pixel pitch of b (mm), conversion is performed so that the same length and width of X2 and Y2 (cm) are secured.

[Equation 1]

$$X2 = Y1 \times (a/b)$$

[Equation 2]

$$Y2 = X1 \times (a/b)$$

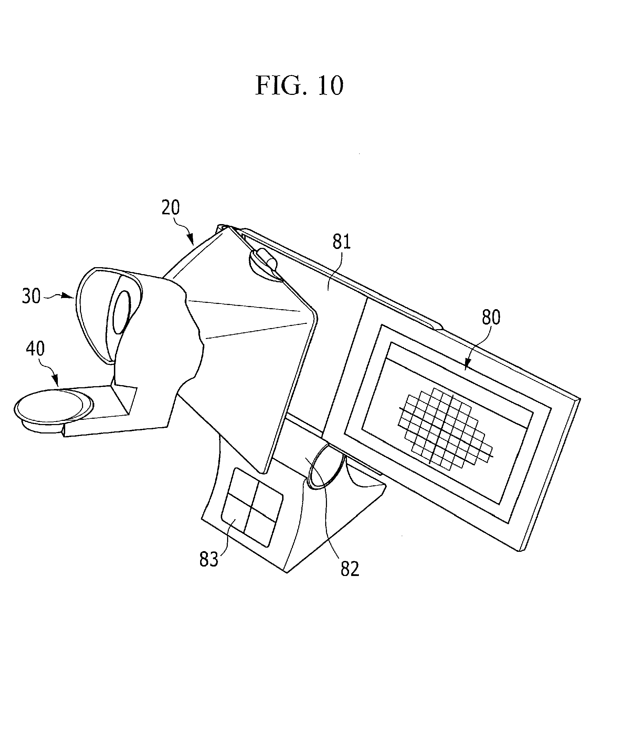

FIG. 10 is an entire schematic diagram of a visual field self-testing system with which a tablet computer is connected according to another exemplary embodiment of the present invention.

Referring to FIG. 10, the system includes a tablet computer 80 presenting a visual field measurement region testing a visual field and a visual field measurement result, a device insertion unit 81 fixing the tablet computer 80, a device angle adjustment unit 82 inclining the tablet computer 80, an examinee information input unit 83 inputting examinee information, the attachable and detachable light blocking unit 20 providing a visual field testing environment, the eye selection unit 30, the face support unit 40, and the visual stimulus confirmation unit. The visual stimulus confirmation unit may be connected to the tablet computer 80.

Guide units are formed at upper and lower edges of the device insertion unit 81 so that the tablet computer 80 is inserted in a sliding mode and fixed. The examinee information input unit 83 may be formed of a bar code reader or an RFID reader to recognize examinee information constituted by the bar code or RFID. The examinee information input unit 83 may be connected to the tablet computer 80 to transfer inputted examinee information to the tablet computer 80.

Meanwhile, as shown in FIG. 10, in the present exemplary embodiment, the eye selection unit 30 and the face support unit 40 may be integrated with each other to be fastened to the attachable and detachable light blocking unit 20. However, as shown in FIG. 1, the eye selection unit 30 and the face support unit 40 may be manufactured as separate parts and assembled with each other to use.

As described above, the visual field measurement region of the system according to the exemplary embodiment of the present invention may be constituted for the purpose of a visual field examination and an ophthalmology examination such as visual acuity, macular degeneration, and color blindness.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims, the detailed description of the invention, and the drawings.

**Claims**

1. A personal computer-based visual field self-testing system comprising:

   a computer monitor connected to a computer main body and presenting a visual field measurement region and a visual field measurement result;
   an attachable and detachable light blocking unit equipped on a front side of the computer monitor to provide a visual field testing environment;
   an eye selection unit equipped on a rear side of the attachable and detachable light blocking unit to block external light and select an examination eye;
   a face support unit including the eye selection unit and supporting a face of an examinee; and
   a visual stimulus confirmation unit connected to the computer main body and operated by the examinee according to a visual stimulus of the visual field measurement region.

2. The personal computer-based visual field self-testing system of claim 1, wherein:

   the computer monitor includes
   a first monitor providing the visual field measurement region testing a visual field and a user interface testing the visual field, and
   a second monitor providing a performed visual field testing result.

3. The personal computer-based visual field self-testing system of claim 1, wherein:

   the attachable and detachable light blocking unit comes into close contact with
   the computer monitor at a position completely including the visual field measurement region displayed in the computer monitor, and
   blocks the external light to the computer monitor.

4. The personal computer-based visual field self-testing system of claim 1, wherein:

   the eye selection unit includes
   a housing surrounding eyes of the examinee to block the external light,
   an examination eye body tube fastened to a side of the housing to come into close contact with an eye of the examinee, which is to be examined,
   a connection unit fastened so that the body tube coincides with a center of the visual field measurement region and connected to a narrow end of the attachable and detachable light blocking unit, and

a fence provided at an outskirt of the examination eye body tube in the housing.

5. The personal computer-based visual field self-testing system of claim 4, wherein:

the eye selection unit is supported by a horizontal setting unit provided on a side of the connection unit.

6. The personal computer-based visual field self-testing system of claim 1, wherein:

the face support unit includes a forehead support member on which a forehead of the examinee is leaned, a height adjustment member adjusting a height of the forehead support member, a chin support member supporting a chin of the examinee, a left and right adjustment member adjusting left and right movement of the chin support member, a height adjustment screw adjusting a height of the chin support member, and a prop fastened by the height adjustment screw to support a face weight of the examinee.

7. The personal computer-based visual field self-testing system of claim 1, wherein:

the visual stimulus confirmation unit includes a left button and a right button pushed by the examinee according to a shape of a central visual stimulus presented in the visual field measurement region, a visual stimulus confirmation button pushed when the presented visual stimulus is displayed, and a connection port connected to the computer main body.

8. The personal computer-based visual field self-testing system of claim 1, wherein:

the visual field measurement region includes a central fixation target, the visual stimuli, and a blind spot visual stimulus constituted to measure a visual field of an eye to be examined.

9. The personal computer-based visual field self-testing system of claim 8, wherein:

the central fixation target is formed to have at least one selected from a numeral, a sign, a letter, a figure, and animal and matter shapes.

10. The personal computer-based visual field self-testing system of claim 1, further comprising:

an examinee information input unit to recognize examinee information constituted by a bar code or a RFID.

11. The personal computer-based visual field self-testing system of claim 1, wherein:

the computer monitor is a tablet computer integrated with the computer main body, the personal computer-based visual field self-testing system further includes a device insertion unit into which the tablet computer is inserted; and a device angle adjustment unit connected to a lower end of the device insertion unit to lean the tablet computer, and the visual stimulus confirmation unit is connected to the tablet computer.

12. The personal computer-based visual field self-testing system of claim 1, wherein:

the visual field measurement region is constituted to selectively perform an ophthalmology examination including visual acuity, macular degeneration, and color blindness.

13. A personal computer-based visual field self-testing method comprising:

an input step of inputting a visual field measurement region, a visual field measurement method, and a central fixation target shape in a computer main body; a visual stimulus presentation step of simultaneously performing presentation of a central fixation target and presentation of a visual stimulus or a blind spot visual stimulus; a first judging step of judging whether an examination of the visual stimulus is finished and performing the visual stimulus presentation step when the examination of the visual stimulus is not finished; a gaze fixing error rate calculation step of calculating a gaze fixing error rate when the examination of the visual stimulus is finished; a second judging step of judging whether the gaze fixing error rate is a set value or less, and performing the input step when the gaze fixing error rate is more than the set value; and a result provision step of providing a visual field measurement result to a computer monitor and finishing a visual field testing program when the gaze fixing error rate is the set value or less.

**14.** The personal computer-based visual field self-testing method of claim 13, wherein:

the input step further includes
a conversion step of automatically grasping a size and a resolution of the computer monitor and converting the visual field measurement region to be the same as a screen of the computer monitor and thus display the converted visual field measurement region.

**15.** The personal computer-based visual field self-testing method of claim 13, wherein:

the visual stimulus presentation step includes presenting a plurality of central fixation targets having different shapes by flickering at the same or different predetermined speeds.

**16.** The personal computer-based visual field self-testing method of claim 13, wherein:

the result provision step further includes presenting a degree of visual field damage progress based on the individually accumulated visual field measurement result.

FIG. 1

FIG. 2

FIG. 3

# FIG. 4

FIG. 5

FIG. 6

## FIG. 7

```
                    ( start )
                        │
                        ▼
        ┌──────────────────────────────┐
        │ select visual field measurement │──ST11 ┐
        └──────────────────────────────┘          │
                        │                          │
                        ▼                          ├ ST10
        ┌──────────────────────────────┐          │
        │ select visual field measurement │──ST12 │
        └──────────────────────────────┘          │
                        │                          │
                        ▼                          │
        ┌──────────────────────────────┐          │
        │ select shape of central fixation target │──ST13 ┘
        └──────────────────────────────┘
```

ST21                                   ST22
```
  ┌─────────────────┐      ┌─────────────────────────┐
  │ present central  │      │ present visual stimulus or │   ┐
  │ fixation target  │      │ Blind spot visual stimulus │   ├ ST20
  └─────────────────┘      └─────────────────────────┘   ┘
```

ST30
```
           ◇ is examination of
             all visual stimuli finished? ◇───── no
                     │
                    yes
                     │
                     ▼
        ┌──────────────────────────────┐
        │ calculate gaze fixing error ratio │──ST40
        └──────────────────────────────┘
```

ST50
```
  no ─── ◇ calculate gaze fixing error ratio
             ≤ 33%? ◇
                     │
                    yes
                     │
                     ▼
        ┌──────────────────────────────┐
        │ calculate gaze fixing error ratio │──ST60
        └──────────────────────────────┘
                     │
                     ▼
                   ( end )
```

# FIG. 8

(A)

611

2

(B)

612

(C)

613

# FIG. 9

(pixel pitch = a mm)

Y1
(cm)

A

X1 (cm)

(pixel pitch = b mm)

Y2
(cm)

A

X2 (cm)

FIG. 10

## INTERNATIONAL SEARCH REPORT

| International application No. |
| --- |
| **PCT/KR2012/011148** |

**A. CLASSIFICATION OF SUBJECT MATTER**

*A61B 3/024(2006.01)i*

According to International Patent Classification (IPC) or to both national classification and IPC

**B. FIELDS SEARCHED**

Minimum documentation searched (classification system followed by classification symbols)
A61B 3/024; A61B 3/113; G10L 15/04; A61H 5/00; A61H 1/02; A61B 5/16; G06T 1/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: view, attention, computer, shading, diagnosis, inspection, select, face, support, check, indication, error, measurement, fixture, determine

**C. DOCUMENTS CONSIDERED TO BE RELEVANT**

| Category* | Citation of document, with indication, where appropriate, of the relevant passages | Relevant to claim No. |
| --- | --- | --- |
| A | JP 2010-526623 A (UNIVERSITY COURT OF THE UNIVERSITY OF EDINBURGH) 05 August 2010<br>See abstract; paragraph [0033]; claim 1; figure 1 | 1-16 |
| A | JP 2008-544793 A (ABERDEEN UNIVERSITY) 11 December 2008<br>See abstract; paragraphs [0034]-[0039]; claim 1; figure 1 | 1-16 |
| A | JP 11-113888 A (FUKUHARA JUN) 27 April 1999<br>See abstract; paragraphs [0013]-[0020]; claim 1; figure 1 | 1-16 |
| A | JP 2009-059257 A (SONY CORP.) 19 March 2009<br>See abstract; paragraphs [0037]-[0062]; claim 1; figure 1 | 1-16 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 06 MARCH 2013 (06.03.2013) | **20 MARCH 2013 (20.03.2013)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br>Telephone No. |
| --- | --- |

Form PCT/ISA/210 (second sheet) (July 2009)

International application No.

**PCT/KR2012/011148**

| Patent document cited in search report | Publication date | Patent family member | Publication date |
|---|---|---|---|
| JP 2010-526623 A | 05.08.2010 | AU 2008-249842 A1 | 20.11.2008 |
| | | CA 2687409 A1 | 20.11.2008 |
| | | CN 101742957 A | 16.06.2010 |
| | | EP 2148609 A1 | 03.02.2010 |
| | | GB 0709405 D0 | 27.06.2007 |
| | | JP 2010-526623 T | 05.08.2010 |
| | | US 2010-0195051 A1 | 05.08.2010 |
| | | WO 2008-139137 A1 | 20.11.2008 |
| JP 2008-544793 A | 11.12.2008 | AT 488210 T | 15.12.2010 |
| | | AU 2006-264688 A1 | 11.01.2007 |
| | | CA 2613223 A1 | 11.01.2007 |
| | | CN 101242798 A | 13.08.2008 |
| | | DE 602006018312 D1 | 30.12.2010 |
| | | EP 1906907 A2 | 09.04.2008 |
| | | EP 1906907 B1 | 17.11.2010 |
| | | GB 0513603 D0 | 10.08.2005 |
| | | JP 2008-544793 T | 11.12.2008 |
| | | RU 2008102875 A | 10.08.2009 |
| | | US 2010-0141894 A1 | 10.06.2010 |
| | | WO 2007-003902 A2 | 11.01.2007 |
| | | WO 2007-003902 A3 | 19.04.2007 |
| | | WO 2007-003902 A3 | 11.01.2007 |
| JP 11-113888 A | 27.04.1999 | EP 0962188 A1 | 08.12.1999 |
| | | EP 0962188 A4 | 21.03.2001 |
| | | JP 04-171995 B2 | 29.10.2008 |
| | | US 6606577 B1 | 12.08.2003 |
| | | WO 99-20180 A1 | 29.04.1999 |
| | | WO 99-20180A1 | 29.04.1999 |
| JP 2009-059257 A | 19.03.2009 | CN 101383000 A | 11.03.2009 |
| | | EP 2031545 A2 | 04.03.2009 |
| | | KR 10-2009-0024086 A | 06.03.2009 |
| | | KR 20090024086 A | 06.03.2009 |
| | | US 2009-0060291 A1 | 05.03.2009 |
| | | US 8295556 B2 | 23.10.2012 |

Form PCT/ISA/210 (patent family annex) (July 2009)